# EUROPÄISCHE PATENTSCHRIFT

(11) **EP 1 470 143 B1**
(45) Veröffentlichungstag und Bekanntmachung des Hinweises auf die Patenterteilung: **31.10.2007**
(21) Anmeldenummer: 03734605.3
(22) Anmeldetag: 31.01.2003
(51) Int. Cl.: C07H 15/203, A61K 31/7034

(54) **VERBINDUNGEN, DIE FAKTOR XA-AKTIV T INHIBIEREN**
COMPOUNDS THAT INHIBIT FACTOR XA ACTIVITY
COMPOSES INHIBANT L'ACTIVITE DU FACTOR XA

(30) Priorität: 31.01.2002 DE 10204072; 03.01.2003 DE 10300049
(43) Veröffentlichungstag der Anmeldung: 27.10.2004
(73) Patentinhaber: Morphochem Aktiengesellschaft Für Kombinatorische Chemie, 81379 München (DE)
(72) Erfinder: ECKL, Robert, 81379 München (DE); SCHABBERT, Silke, 81379 München (DE); FUCHS, Thilo, 81379 München (DE); WEBER, Lutz, 82110 Germering (DE); OEFNER, Christian, 79108 Freiburg (DE)
(74) Vertreter: Forstmeyer, Dietmar
(86) Internationale Anmeldenummer: PCT/EP2003/001011
(87) Internationale Veröffentlichungsnummer: WO 2003/064440

(56) Entgegenhaltungen:
- EP-A- 0 290 321
- EP-A- 0 560 568
- EP-A- 0 921 116
- EP-A- 1 078 917
- WO-A-02/16312
- WO-A-02/42270
- WO-A-99/65934

## Beschreibung

Die vorliegende Erfindung betrifft neue Verbindungen mit blutgerinnungshemmender Wirkung (sogenannte Antikoagulantien) sowie ihre pharmakologisch akzeptablen Salze bzw. Solvate und Hydrate, pharmazeutische Zusammensetzungen, die diese als Wirkstoff enthalten, Verfahren zur Herstellung solcher Verbindungen, Salze und Zusammensetzungen sowie deren Verwendung zur Vorbeugung und/oder Behandlung von thromboembolytischen Erkrankungen. Diese Verbindungen, Salze und Zusammensetzungen stellen sehr wirksame Faktor Xa-Inhibitoren dar. Die vorliegende Erfindung betrifft auch Pro-Drugs, optisch aktive Formen, Racemate und Diastereomere dieser Verbindungen und Salze.

Thromboembolytische Erkrankungen beruhen auf einer erhöhten Blutgerinnungsneigung bei Personen mit Risikofaktoren, wie z.B. größeren Operationen, längerer Immobilisierung, Knochenbrüchen der unteren Extremitäten, Fettleibigkeit, Blutfett-Stoffwechselstörungen, Infektionen mit gramnegativen Organismen, Krebs und höherem Alter.

Venöse Thrombosen können dazu führen, daß das von der betroffenen Vene entsorgte Gewebe ein Ödem oder eine Entzündung entwickelt. Thrombose einer tieferliegenden Vene (sogenannte "Deep Vein Thrombosis") kann zu schwerwiegenden Komplikationen wie z.B. Lungenembolie führen. Arterielle Thrombose kann zur ischämischen Nekrose des von der betroffenen Arterie versorgten Gewebes führen, wie z.B. zu myokadialem Infarkt im Falle einer betroffenen Herzkranzarterie. Weitere thromboembolytische Erkrankungen sind z.B. Arteriosklerose, Apoplexie (Schlaganfall), Angina pectoris, Claudicatio intermittens.

Unter normalen physiologischen Bedingungen schützt die natürliche Blutgerinnung vor größerem Blutverlust aus einem beschädigten Blutgefäß. Bei der Blutgerinnung erfolgt eine Umwandlung des flüssigen Blutes in den Blutkuchen, eine gallertartige Masse, die die Abdichtung verletzter Blutgefäße durch Pfropfbildung bewirkt. Dabei erfolgt die Umwandlung des im Plasma vorhandenen löslichen Fibrinogens in den faserig-gallertartigen Gerinnungsstoff, das Fibrin, in einem mehrstufigen Prozeß, der sogenannten Blutgerinnungskaskade.

Man unterscheidet zwischen zwei verschiedenen Wegen der Aktivierung der Blutgerinnung. Der intrinsische Blutgerinnungsweg wird eingeleitet, wenn Blut mit unphysiologischen Oberflächen in Berührung kommt. Der extrinsische Blutgerinnungsweg wird durch die Verletzung von Blutgefäßen eingeleitet. Beide Blutgerinnungswege münden in einem gemeinsamen Weg, in dem der Blutgerinnungsfaktor X, eine Serin-Proteinase, in seine aktive Form (Faktor Xa) überführt wird. Faktor Xa bewirkt zusammen mit Faktor Va und Ca²⁺ im sogenannten Prothrombinasekomplex, daß Prothrombin in Thrombin überführt wird, welches seinerseits durch Abspaltung von Peptiden aus Fibrinogen Fibrin-Monomere freisetzt, die in der Lage sind, zu Fibrinfasern zu koagulieren. Durch den Faktor XIII kommt es schließlich zur Quervernetzung und somit Stabilisierung der Fibrinfasern.

Antikoagulantien kommen sowohl zur Vorbeugung als auch zur Behandlung von thromboembolytischen Erkrankungen zum Einsatz. Man unterscheidet bei den Antikoagulantien im engeren Sinne das sofort wirksame Heparin, welches direkt bestimmte Faktoren der Blutgerinnung hemmt, von den Vitamin K-Antagonisten (z.B. Cumarin-Derivate). Letztere hemmen die von der Anwesenheit von Vitamin K abhängige Produktion bestimmter Gerinnungsfaktoren in der Leber und setzen mit ihrer Wirkung erst langsam ein. Weitere gerinnungshemmende Mittel sind die Fibrinolytika, die eine direkte oder indirekte Aktivierung des fibrinolytischen Systems hervorrufen, und Thrombozyten-Aggregationshemmer wie z.B. Acetylsalicylsäure. Ein seltener eingesetztes Verfahren ist die Senkung des Fibrinogenspiegels im Blut durch das Enzym Ancrod. Das Ziel der Anwendung gerinnungshemmender Mittel ist, die Entstehung eines gefäßverschließenden Blutgerinnsels zu verhüten oder auch es nach seiner Bildung wieder aufzulösen.

Die oben genannten Antikoagulantien im engeren Sinne, d.h. Heparin und Vitamin K-Antagonisten, weisen Nachteile auf. Beim Heparin unterscheidet man unfraktioniertes Heparin (UFH) und Heparin mit niedrigem Molekulargewicht (LMWH). Nachteilig bei UFH ist die Tatsache, daß es in der Regel intravenös verabreicht werden muß, eine variierende blutgerinnungshemmende Wirkung aufweist und somit häufige Überwachungen des Patienten und Dosisanpassungen erforderlich macht. LMWH kann zwar in konstanter, unüberwachter Dosierung subkutan zum Einsatz kommen, weist aber aufgrund seiner geringen Kettenlänge eine gegenüber UFH stark verringerte Wirkung auf.

Die Vitamin K-Antagonisten wie z.B. Warfarin zeigen - vermutlich genetisch bedingt - eine von Patient zu Patient unterschiedliche Wirksamkeit. Neben dem oben erwähnten langsamen Einsetzen der Wirkung ist dies mit dem Nachteil verbunden, daß die Patienten überwacht werden müssen und eine individuelle Dosisanpassung erforderlich ist.

Weitere bekannte Antikoagulantien gehören der Gruppe der Thrombin-Inhibitoren an. Aktuelle Übersichten der einschlägigen Forschungstätigkeiten auf diesem Gebiet finden sich z.B. bei Jules A. Shafer, Current Opinion in Chemical Biology, 1988, 2: 458-485, Joseph P. Vacca, Current Opinion in Chemical Biology, 2000, 4: 394-400 sowie Fahad Al-Obeidi und James A. Ostrem, DDT, Bd. 3, Nr. 5, Mai 1998: 223-231.

Ein entscheidender Nachteil der Thrombin-Inhibitoren besteht darin, daß zur Erzielung der gewünschten Wirkung eine derartig starke Unterdrückung der Thrombin-Aktivität in vivo erforderlich ist, daß sich die Blutungsneigung erhöhen kann, was die Dosierung erschwert.

Demgegenüber bewirken Faktor Xa-Inhibitoren eine Unterdrückung der Neubildung von Thrombin aus Prothrombin, während sie eine vorhandene Thrombin-Aktivität, die für eine primäre Hämostase erforderlich ist, nicht beeinträchtigen.

Die Wirkungs- und Nebenwirkungsspektren dieser Faktor Xa-Inhibitoren sind zum Teil noch nicht vollständig untersucht.

Eine Aufgabe der vorliegenden Erfindung bestand in der Bereitstellung neuer Verbindungen mit nützlichen Eigenschaften, insbesondere blutgerinnungshemmender Wirkung.

Genauer gesagt bestand die Aufgabe in der Bereitstellung neuer Faktor Xa-Inhibitoren mit verbesserter Wirksamkeit, verringerter Nebenwirkung und/oder erhöhter Selektivität. Zudem sollten geeignete pharmazeutische Zusammensetzungen bereitgestellt werden. Diese Verbindungen bzw. Zusammensetzungen sollten bevorzugt parenteral oder oral, insbesondere oral verabreichbar sein.

Eine weitere Aufgabe der vorliegenden Erfindung bestand in der Bereitstellung eines Verfahrens zur Herstellung dieser neuen Verbindungen.

Des weiteren sollten diese neuen Verbindungen zur Vorbeugung und/oder Behandlung von thromboembolytischen Erkrankungen geeignet sein.

Die vorliegende Erfindung beschreibt blutgerinnungshemmende Verbindungen, deren pharmakologisch akzeptable Salze bzw. Solvate und Hydrate und Formulierungen, die eine hohe Aktivität und Selektivität aufweisen und die insbesondere oral verabreicht werden können. Die vorliegende Erfindung betrifft des weiteren Pro-Drugs, optisch aktive Formen, Racemate und Diastereomeren dieser Verbindungen und Salze. Die besagten Verbindungen und Salze können auch ihrerseits Pro-Drugs sein, die erst durch Metabolisierung aktiviert werden. Ebenfalls beschrieben werden pharmazeutische Zusammensetzungen, die die besagten Verbindungen bzw. Salze etc. als Wirkstoff enthalten.

Gegenstand der vorliegenden Erfindung ist eine Verbindung der allgemeinen Formel (I) gemäß Anspruch 1.

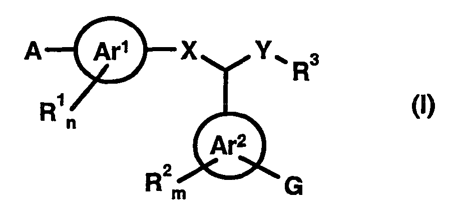

Die nachfolgenden Definitionen beziehen sich auf die gesamte Beschreibung und insbesondere auf die Ansprüche:

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1, 2, 3, 4, 5 oder 6 Kohlenstoffatome aufweist, z.B. die Methyl-, Ethyl-, Propyl-, Isopropyl-, Isobutyl-, tert-Butyl, n-Hexyl-, 2,2-Dimethylbutyl- oder n-Octyl-Gruppe.

Der Ausdruck Alkenyl bezieht sich auf geradkettige oder verzweigte Kohlenwasserstoffgruppen mit mindestens einer Doppelbindung, die 2 bis 20 Kohlenstoffatome, vorzugsweise 2 bis 12 Kohlenstoffatome, besonders bevorzugt 2, 3, 4, 5 oder 6 Kohlenstoffatome aufweisen, z. B. die Ethenyl-, Allyl-, Isoprenyl- oder Hex-2-enyl-Gruppe. Bevorzugt weisen sie eine oder zwei (besonders bevorzugt eine) Doppelbindungen auf.

Der Ausdruck Alkinyl bezieht sich auf geradkettige oder verzweigte Kohlenwasserstoffgruppen mit mindestens einer Dreifachbindung, die 2 bis 20 Kohlenstoffatome, vorzugsweise 2 bis 12 Kohlenstoffatome, besonders bevorzugt 2, 3, 4, 5 oder 6 Kohlenstoffatome aufweisen, z.B. die Acetylenyl- oder Propargyl-Gruppe. Bevorzugt weisen sie eine oder zwei (besonders bevorzugt eine) Dreifachbindungen auf.

Des weiteren beziehen sich die Begriffe Alkyl, Alkenyl und Alkinyl auf Gruppen, bei der ein, zwei, drei oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome unabhängig voneinander durch ein Sauerstoff-, Stickstoff-, Phosphor-, Bor-, Selen-, Silizium- und/oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe wie z. B. Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁, C₂, C₃, C₄, C₅ oder C₆-Alkyl-, eine C₂, C₃, C₄, C₅ oder C₆-Alkenyl- oder eine C₂, C₃, C₄, C₅ oder C₆ -Alkinylgruppe; R^{b} ein Wasserstoffatom, eine C₁, C₂, C₃, C₄, C₅ oder C₆-Alkyl-, eine C₂, C₃, C₄, C₅ oder C₆ -Alkenyl- oder eine C₂, C₃, C₄, C₅ oder C₆-Alkinylgruppe; R^{c} ein Wasserstoffatom, eine C₁, C₂, C₃, C₄, C₅ oder C₆ -Alkyl-, eine C₂ C₃, C₄, C₅ oder C₆ -Alkenyl- oder eine C₂, C₃, C₄, C₅ oder C₆ -Alkinylgruppe; R^{d} ein Wasserstoffatom, eine C₁, C₂, C₃, C₄, C₅ oder C₆ -Alkyl-, eine C₂, C₃, C₄, C₅ oder C₆-Alkenyl- oder eine C₂ C₃, C₄, C₅ oder C₆-Alkinylgruppe und Y^{a} eine Bindung, eine C₁, C₂, C₃, C₄, C₅ oder C₆ -Alkylen-, eine C₂, C₃, C₄, C₅ oder C₆ -Alkenylen- oder eine C₂ C₃, C₄, C₅ oder C₆-Alkinylengruppe ist, wobei jede Heteroalkylgruppe mindestens ein Kohlenstoffatom enthält und ein, zwei, drei oder mehrere Wasserstoffatome durch Halogenatome (insbesondere Fluor- oder Chloratome) ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methylaminomethyl, Ethylaminomethyl, Diiso-Propylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat, Carbonyl und Alkylnitrilgruppen.

Der Ausdruck Cycloalkyl bezieht sich auf eine gesättigte cyclische Gruppe, die einen oder mehrere Ringe (bevorzugt 1 oder 2, besonders bevorzugt 1) aufweist, welche insgesamt 3 bis 14 Ring-Kohlenstoffatome, vorzugsweise 3 bis 10 (insbesondere 3, 4, 5, 6 oder 7) Ring-Kohlenstoffatome enthalten. Der Ausdruck Cycloalkyl bezieht sich weiterhin auf entsprechende Gruppen, bei denen ein oder mehrere Wasserstoffatome unabhängig voneinander durch Fluor-, Chlor-, Brom- oder Jodatome oder OH, =O, SH, =S, NH₂, =NH oder NO₂-Gruppen ersetzt sind, also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]decanyl-, Norborny-, Cyclohexyl-, Cyclopentenyl-, Cyclohexadienyl-, Decalinyl-, Cubanyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome unabhängig voneinander durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2, (besonders bevorzugt 1) Ringe mit 3 bis 10 (insbesondere 3, 4, 5, 6 oder 7) Ringatomen. Der Ausdruck Heterocycloalkyl bezieht sich weiterhin auf entsprechende Gruppen, bei denen ein oder mehrere Wasserstoffatome unabhängig voneinander durch Fluor-, Chlor-, Brom- oder Jodatome oder OH, = O, SH, =S, NH₂, =NH oder NO₂-Gruppen ersetzt sind. Beispiele sind die Piperidyl-, Morpholinyl-, Urotropinyl-, Pyrrolidinyl-, Tetrahydrothiophenyl-, Tetrahydropyranyl-, Tetrahydrofuryl-, Oxacyclopropyl-, Azacyclopropyl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten. Bevorzugt enthält eine Alkylcycloalkylgruppe eine oder zwei (bevorzugt eine) Cycloalkylgruppen, die jeweils 3 bis 10 (insbesondere 3, 4, 5, 6 oder 7) Ring-Kohlenstoffatome enthalten, und eine oder zwei Alkyl-, Alkenyl- oder Alkinylgruppen mit 1 oder 2 bis 6 Kohlenstoffatomen.

Beispiele derartiger Verbindungen sind:
Alkylcycloalkyl, Alkyldicycloalkyl, Dialkylcycloalkyl, Alkylcycloalkenyl, Alkyldicycloalkenyl, Dialkylcycloalkenyl, Alkenylcycloalkyl, Alkenyldicycloalkyl, Dialkyldicycloalkyl, Alkenylcycloalkenyl, Alkenyldicycloalkenyl, Dialkyldicycloalkyl, Dialkenylcycloalkyl, Dialkenylcycloalkenyl, Alkinylcycloalkyl, Alkinyldicycloalkyl, Dialkenyldicycloalkyl, Alkinylcycloalkenyl, Alkinyldicycloalkenyl, Dialkenyldicycloalkenyl.

Der Ausdruck Heteroalkylcycloalkyl bezieht sich auf Alkylcycloalkylgruppen, wie oben definiert, in denen ein oder mehrere (bevorzugt 1, 2 oder 3) Kohlenstoffatome unabhängig voneinander durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 (bevorzugt einen) Ringe mit jeweils 3 bis 10 (insbesondere 3, 4, 5, 6 oder 7) Ringatomen und eine oder zwei Alkyl, Alkenyl, Alkinyl oder Heteroalkylgruppen mit jeweils 1 oder 2 bis 6 Kohlenstoffatomen. Beispiele derartiger Gruppen, die durch die Alkyl-, Alkenyl-, Alkinyl- und/oder Heteroalkylgruppen substituiert sein können, sind
Heteroalkylcycloalkyl, Heteroalkyldicycloalkyl, Diheteroalkylcycloalkyl, Heteroalkylcycloalkenyl, Heteroalkyldicycloalkenyl, Diheteroalkylcycloalkenyl, Heteroalkenylcycloalkyl, Heteroalkenyldicycloalkyl, Diheteroalkyldicycloalkyl, Heteroalkenylcycloalkenyl, Heteroalkenyldicycloalkenyl, Diheteroalkyldicycloalkyl, Diheteroalkenylcycloalkyl, Diheteroalkenylcycloalkenyl, Heteroalkinylcycloalkyl, Heteroalkinyldicycloalkyl, Diheteroalkenyldicycloalkyl, Heteroalkinylcycloalkenyl, Heteroalkinyldicycloalkenyl, Diheteroalkenyldicycloalkenyl,
Alkylheterocycloalkyl, Alkyldiheterocycloalkyl, Dialkylheterocycloalkyl, Alkylheterocycloalkenyl, Alkyldiheterocycloalkenyl, Dialkylheterocycloalkenyl, Alkenylheterocycloalkyl, Alkenyldiheterocycloalkyl, Dialkyldiheterocycloalkyl, Alkenylheterocycloalkenyl, Alkenyldiheterocycloalkenyl, Dialkyldiheterocycloalkyl Dialkenylheterocycloalkyl, Dialkenylheterocycloalkenyl, Alkinylheterocycloalkyl, Alkinyldiheterocycloalkyl, Dialkenyldiheterocycloalkyl, Alkinylheterocycloalkenyl, Alkinyldiheterocycloalkenyl, Dialkenyldiheterocycloalkenyl,
Heteroalkylheterocycloalkyl, Heteroalkyldiheterocycloalkyl, Diheteroalkylheterocycloalkyl, Heteroalkylheterocycloalkenyl, Heteroalkyldiheterocycloalkenyl, Diheteroalkylheterocycloalkenyl, Heteroalkenylheterocycloalkyl, Heteroalkenyldiheterocycloalkyl, Diheteroalkyldiheterocycloalkyl, Heteroalkenylheterocycloalkenyl, Heteroalkenyldiheterocycloalkenyl, Diheteroalkyldiheterocycloalkyl, Diheteroalkenylheterocycloalkyl, Diheteroalkenylheterocycloalkenyl, Heteroalkinylheterocycloalkyl, Heteroalkinyldiheterocycloalkyl, Diheteroalkenyldiheterocycloalkyl, Heteroalkinylheterocycloalkenyl, Heteroalkinyldiheterocycloalkenyl, Diheteroalkenyldiheterocycloalkenyl,

Besonders bevorzugt sind:
Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe, bevorzugt einen Ring aufweist, der 6 bis 14 Ring-Kohlenstoffatome, vorzugsweise 6 bis 10 (insbesondere 6) Ring-Kohlenstoffatome enthält. Der Ausdruck Aryl (bzw. Ar) bezieht sich weiterhin auf entsprechende Gruppen, bei denen ein oder mehrere Wasserstoffatome unabhängig voneinander durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe, bevorzugt einen Ring aufweist, die 5 bis 14 Ringatome, vorzugsweise 5, 6, 7, 8, 9 oder 10 (insbesondere 5 oder 6) Ringatome enthalten, wobei ein oder mehrere (bevorzugt 1, 2, 3 oder 4) Ringatome Sauerstoff-, Stickstoff-, Phosphor- oder Schwefel-Ringatome (bevorzugt O, S oder N) sind. Der Ausdruck Heteroaryl bezieht sich weiterhin auf entsprechende Gruppen, bei denen ein oder mehrere Wasserstoffatome unabhängig voneinander durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-(z. B. 6-Indolyl-), Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- als auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Arylcycloalkinyl, Alkylarylcycloalkyl- Alkylarylcycloalkenyl-Alkylarylcycloalkinyl-, Alkylarylalkyl-, Alkylarylalkenyl-, Alkylarylalkinyl-, Alkenylarylalkenyl-, Alkinylarylalkenyl-Alkinylarylalkinyl- und Arylalkylcycloalkylgruppen. Konkrete Beispiele für Aralkyle sind Toluol, Trityl, Xylol, Mesitylen, Styrol, Benzylchlorid, o-Fluortoluol, 1H-Inden, Tetralin, Dihydro-naphthaline, Indanon, Phenylcyclopentyl, Cumol, Cyclohexylphenyl, Fluoren und Indan. Bevorzugt enthält eine Aralkylgruppe ein aromatisches Ringsystem (1 oder 2 Ringe) mit 6 bis 10 Kohlenstoffatomen (z.B. Phenyl- oder Naphthyl-) und ein oder zwei Alkyl-, Alkenyl- und/oder Alkinylgruppen mit jeweils 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome unabhängig voneinander durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- als auch Alkyl-, Alkenyl-, oder Alkinyl- und/oder Heteroalkyl- und/oder Cycloalkyl- oder Cycloalkenyl-, und/oder Heterocycloalkyl- oder Heterocycloalkenylgruppen enthalten. Bevorzugt enthält eine Heteroaralkylgruppe ein oder zwei aromatische Ringsysteme (mit jeweils 1 oder 2 Ringen) mit jeweils 5, 6, 7, 8, 9 oder 10 Ring-Kohlenstoffatomen und ein oder zwei Alkyl-, Alkenyl- und/oder Alkinylgruppen mit 1 oder 2, 3, 4, 5 oder 6 Kohlenstoffatomen und/oder eine Cycloalkylgruppe mit 5 oder 6 Ringkohlenstoffatomen, wobei 1, 2, 3 oder 4 dieser Kohlenstoffatome und/oder Ring-Kohlenstoffatome unabhängig voneinander durch Sauerstoff-, Schwefel- oder Stickstoffatome ersetzt sind.

Beispiele sind Aryl-heteroalkyl-, Aryl-heterocycloalkyl-, Aryl-heterocycloalkenyl-, Aryl-alkyl-heterocycloalkyl-, Aryl-alkenyl-heterocycloalkyl-, Aryl-alkinylheterocycloalkyl-, Aryl-alkyl-heterocycloalkenyl-, Aryl-heteroalkyl-heterocycloalkyl, Heteroaryl-alkyl-, Heteroaryl-alkenyl-, Heteroaryl-alkinyl-, Heteroaryl-heteroalkyl-, Heteroaryl-cycloalkyl-, Heteroarylcycloalkenyl-, Heteroaryl-heterocycloalkyl-, Heteroarylheterocycloalkenyl-, Heteroaryl-alkyl-cycloalkyl-, Heteroaryl-heteroalkyl-cycloalkyl-, Heteroaryl-alkyl-heterocycloalkenyl-, Heteroaryl-heteroalkyl-cycloalkenyl- und Heteroaryl-heteroalkyl-heterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Aryl, Heterocycloalkyl, Alkylcycloalkyl, Heteroalkylcycloalkyl, Heteroaryl, Aralkyl und Heteroaralkyl beziehen sich auch auf Gruppen, in denen ein oder mehrere Wasserstoffatome unabhängig voneinander durch Fluor-, Chlor-, Brom- oder Jodatome oder OH, =O, SH, =S, NH₂, =NH oder NO₂-Gruppen ersetzt sind (wobei die =0, =S und =NH-Gruppen jeweils zwei Wasserstoffatome ersetzen).

Der Ausdruck "gegebenenfalls substituiert" bezieht sich auf Gruppen, in denen ein, zwei oder mehrere Wasserstoffatome durch Fluor-, Chlor-, Brom- oder Jodatome oder OH, =O, SH, =S, SO₂NH₂, NH₂, =NH oder NO₂-Gruppen ersetzt sind. Dieser Ausdruck bezieht sich weiterhin auf Gruppen, in denen ein, zwei oder mehrere Wasserstoffatome unabhängig voneinander druch unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen ersetzt sind.

In der vorliegenden Anmeldung bezieht sich der Ausdruck Glycosylgruppe bzw. Glycosylrest auf ein über eine α- oder β-O, S, N oder C-glycosidische Bindung (bevorzugt eine O-glycosidische Bindung) gebundenes Saccharid (Mono- oder Oligosaccharid inkl. Aminozucker und N-Acetylaminozuckern), bei dem die OH-Gruppen gegebenenfalls durch Acetyl oder Benzoylgruppen geschützt sein können, insbesondere ein Monosaccharid (z. B. Glucose, Galactose, Fructose, Fucose, Ribose, Glucosamin, N-Acetylglucosamin, Galactosamin, N-Acetylgalactosamin oder Mannose), vorzugsweise β-D-Glucose.

Bevorzugt sind Verbindungen der allgemeinen Formel (I), wobei Y eine Gruppe der Formel CONR⁶ und R³ keine Gruppe der Formel -CHR⁷-CO-NR⁸R⁹ ist, wobei R⁷, R⁸ und R⁹ unabhängig voneinander ein Wasserstoffatom, eine Alkyl-, Alkenyl-Alkinyl-, Heteroalkyl-, Heteroaralkyl-, Heteroaryl-, Aralkyl-, Cycloalkyl-, Heterocycloalkyl-, Alkylcycloalkyl-, Heteroalkylcycloalkyl-, oder eine Arylgruppe sind oder R⁸ und R⁹ zusammen Teil eines Heterocycloalkyl- oder Heteroarylringsystems sind.

Des weiteren bevorzugt sind Verbindungen der allgemeinen Formel (I), wobei Y eine Gruppe der Formel CO, und R³ keine Gruppe der Formel -NR¹⁰-CHR⁷-CO-NR⁸R⁹ ist, wobei R⁷, R⁸, R⁹ und R¹⁰ unabhängig voneinander ein Wasserstoffatom, eine Alkyl-, Alkenyl-, Alkinyl-, Heteroalkyl-, Heteroaralkyl-, Heteroaryl-, Alkylcycloalkyl-, Heteroalkyl-cycloalkyl-, Aralkyl-, Cyclo-alkyl-, Heterocycloalkyl-, oder eine Arylgruppe sind oder R⁸ und R⁹ und/oder R⁷ und R¹⁰ zusammen Teil eines Heterocycloalkyl- oder Heteroarylringsystems sind. Insbesondere sind Verbindungen ausgenommen, bei denen der Rest -Y-R³ die folgende Struktur aufweist:
-C(=O)-NR-CR'R"-C(=O)-N(R"')R^{IV} wobei die Reste R, R', R", R"' und R^{IV} beliebig definiert sind, bzw. beliebige chemische Reste darstellen.

Wiederum bevorzugt ist Ar¹ kein Phenylring, an den A und X in para-Stellung zueinander gebunden sind, wenn A eine Gruppe der Formel -C(=NR⁴)NH₂, (insbesondere -C(=NH)NH₂ ist.

Weiter bevorzugt ist A eine Gruppe der Formel -C(=NR⁴)NH₂.

Des weiteren bevorzugt ist A ein Wasserstoffatom.

Wiederum bevorzugt ist R⁴ ein Wasserstoffatom, eine Hydroxy- oder eine C₁, C₂, C₃ oder C₄-Alkyloxygruppe; besonders bevorzugt ist R⁴ ein Wasserstoffatom.

Weiter bevorzugt ist Ar¹ eine Phenyl- oder eine Heteroarylgruppe mit 5, 6, 7, 8, 9 oder 10 Ringatomen und 1, 2, 3 oder 4 Heteroatomen, die aus O, S und N ausgewählt sind; besonders bevorzugt ist Ar¹ eine Phenylgruppe; insbesondere eine Phenylgruppe, an die die Gruppen A und X in meta-Position zueinander gebunden sind.

Des weiteren bevorzugt ist Ar² eine Phenyl- oder eine Heteroarylgruppe mit 5 oder 6 Ringatomen und 1, 2 oder 3 Heteroatomen, ausgewählt aus O, S und N; besonders bevorzugt ist Ar² eine Phenylgruppe.

Wiederum bevorzugt ist X eine Gruppe der Formel NH, NMe, oder NAc; besonders bevorzugt ist X eine NH-Gruppe.

Des weiteren bevorzugt ist n gleich 0, 1 oder 2; besonders bevorzugt 0 oder 1.

Weiter bevorzugt ist R¹ eine Hydroxygruppe, die für Ar¹ gleich Phenyl insbesondere in para-Stellung zu A gebunden ist.

Wiederum bevorzugt ist m gleich 0 oder 1, wobei die Reste R² und G bevorzugt in ortho-Position zueinander stehen; besonders bevorzugt ist m gleich 0.

Weiter bevorzugt ist Y eine Gruppe der Formel CONH.

Des weiteren bevorzugt ist R³ eine Gruppe der Formel -U-V-W, wobei U eine gegebenenfalls substituierte Arylengruppe mit 6 - 10 der 12 Ring-Kohlenstoffatomen oder eine gegebenenfalls substituierte Heteroarylengruppe mit 5, 6, 7, 8, 9 oder 10 Ring-Kohlenstoffatomen und 1, 2, 3 oder 4 (vorzugsweise 1 oder 2) Heteroatomen, die aus O, S und N ausgewählt sind ist; V eine Bindung, ein Sauerstoffatom, ein Schwefelatom, eine Gruppe der Formel NR¹¹ (wobei R¹¹ ein Wasserstoffatom, eine C₁, C₂, C₃ oder C₄-Alkyl-, eine C₁, C₂, C₃ oder C₄-Heteroalkyl-, eine C₇, C₈, C₉, C₁₀, C₁₁ oder C₁₂-Aralkylgruppe oder eine C₆ C₇, C₈, C₉, C₁₀, C₁₁ oder C₁₂-Heteroaralkylgruppe ist), CO, SO, SO₂ oder SO₂NH ist und W ein Wasserstoffatom, ein C₁-C₄ Alkyl-, C₂-C₄ Alkenyl-, C₂-C₄ Alkinyl-, C₁-C₄ Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist.

Besonders bevorzugt ist U eine gegebenenfalls substituierte Phenylengruppe; insbesondere eine para-Phenylengruppe.

Des weiteren besonders bevorzugt ist V eine Bindung oder eine Carbonylgruppe.

Wiederum besonders bevorzugt ist W eine C₁, C₂, C₃ oder C₄-Alkylgruppe, eine C₁, C₂, C₃ oder C₄-Heteroalkylgruppe mit einem oder zwei O, N oder S-Atomen, eine gegebenenfalls substituierte Phenylgruppe, eine gegebenenfalls substituierte C₃, C₄, C₅, C₆ oder C₇ Cycloalkylgruppe, eine gegebenenfalls substituierte Heterocycloalkylgruppe mit 3-7 (bevorzugt 5 oder 6) Ring-Kohlenstoffatomen und 1, 2 oder 3 Ring-Heteroatomen (unabhängig voneinander ausgewählt aus O, S und N) oder eine gegebenenfalls substituierte Heteroarylgruppe mit 5 oder 6 Ring-Kohlenstoffatomen und 1, 2, 3 oder 4 Ring-Heteroatomen, die aus O, S und N ausgewählt sind.

Bevorzugt sind Verbindungen der allgemeinen Formel I wobei Y eine Gruppe der Formel CONR⁶ ist und R³ keine Gruppe der Formel -CHR⁷-CO-NR⁸R⁹ ist wobei R⁷, R⁸ und R⁹ so definiert sind, wie R⁵, R⁶ und R⁷ in der PCT-Anmeldung PCT/EP 02/01934 (WO 02/068390) der Firma Morphochem AG vom 22. Februar 2002 oder wobei R⁷, R⁸ und R⁹ so definiert sind, wie R⁵, R⁶ und R⁷ in der PCT-Anmeldung PCT/EP 01/09753 (WO 02/16312) der Firma Morphochem AG vom 23. August 2001.

Insbesondere bevorzugt sind Verbindungen der Formel (II): 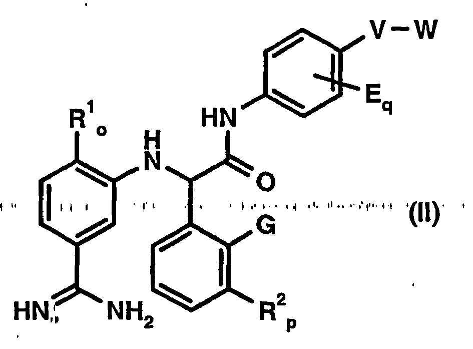 wobei E ein Wasserstoff-, Fluor-, Chlor- oder Bromatom ist und die Reste R¹, R², G, V und W wie oben definiert sind, o gleich 0 oder 1 ist, p gleich 0 oder 1 ist und q gleich 0, 1 oder 2 ist. Besonders bevorzugt ist R¹ eine Hydroxygruppe, R² eine Methoxy- oder eine Ethoxygruppe, G eine β-D-Glucosyloxygruppe, V eine Bindung oder eine Carbonylgruppe (C=O).

Besonders bevorzugt ist W eine cyclische Gruppe der Formel -N(CH₂CH₂)₂Q, wobei Q ein Sauerstoffatom oder eine Gruppe der Formel NR¹² ist, wobei R¹² ein Wasserstoffatom, ein C₁, C₂, C₃ oder C₄-Alkyl- oder ein C₁, C₂, C₃ oder C₄-Heteroalkylrest (z. B. eine Gruppe der Formel -C(=N)NH₂) oder -C(=N)CH₃) ist.

Weiter bevorzugt sind Verbindungen der Formel (I) (mit U = Phenyl) oder (II), wobei V eine Bindung und W ein in ortho-Stellung zu V durch eine Gruppe der Formel SO₂NH₂ oder SO₂Alkyl substituierte Phenylengruppe ist.

Des weiteren bevorzugt sind Verbindungen der Formel (I), wobei A und Ar¹ zusammen eine Indolgruppe sind, an die die Gruppe X bevorzugt in 6-Position gebunden ist.

Wiederum bevorzugt sind Verbindungen der Formel (I), wobei n gleich 0, A und Ar¹ zusammen eine 6-Indolylgruppe, X gleich CONH, m gleich 0, Ar² ein Phenylrest, Y gleich CO und R³ eine Heterocycloalkyl oder eine Heteroalkylcycloalkylgruppe (insbesondere eine Gruppe der Formel -N(CH₂CH₂)₂CH-CH(CH₂CH₂)₂NMe) ist; besonders bevorzugt ist hier die Stereochemie an der Phenylglycineinheit (R).

Verbindungen der Formel (I) oder (II) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) oder (II) sowie Gemische davon umfasst. Des weiteren sind von der vorliegenden Erfindung alle tautomeren Formen der Verbindungen der Formel (I) oder (II) umfasst.

Beispiele für pharmakologisch akzeptable Salze der Verbindungen der Formel (I) oder (II) sind Salze von physiologisch akzeptablen Mineralsäuren wie Salzsäure, Schwefelsäure und Phosphorsäure; oder Salze von organischen Säuren wie Methansulfonsäure, p-Toluolsulfonsäure, Milchsäure, Ameisensäure, Essigsäure, Trifluoressigsäure, Zitronensäure, Bernsteinsäure, Fumarsäure, Maleinsäure und Salicylsäure. Verbindungen der Formel (I) oder (II) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) oder (II) auftreten.

Die pharmazeutischen Zusammensetzungen gemäß der vorliegenden Erfindung enthalten mindestens eine Verbindung der Formel (I) oder (II) als Wirkstoff und fakultativ Trägerstoffe und/oder Adjuvantien.

Die Pro-Drugs (z. B. R. B. Silverman, Medizinische Chemie, VCH Weinheim, 1995, Kapitel 8, S. 361ff), die ebenfalls Gegenstand der vorliegenden Erfindung sind, bestehen aus einer Verbindung der Formel (I) oder (II) und mindestens einer pharmakologisch akzeptablen Schutzgruppe, die unter physiologischen Bedingungen abgespalten wird, z.B. einer Hydroxy, Alkoxy-, Aralkyloxy-, Acyl- oder Acyloxy-Gruppe, wie z.B. einer Methoxy-, Ethoxy-, Benzyloxy-, Acetyl- oder Acetyloxy-Gruppe.

Die hier beschriebenen Verbindungen der Formeln (I) und (II) können nach an sich bekannten Verfahren hergestellt werden. Erfindungsgemäße Verbindungen der Formel (I) und (II) können z. B. durch Umsetzung von Verbindungen der Formeln (III) (gegebenenfalls als Hydrochlorid oder ähnliches Salz), (IV) und (V) über eine Multikomponentenreaktion hergestellt werden (A. Dömling, I. Ugi, Angew. Chem. 2000, 112, 3300-3344), wobei die Reste wie oben definiert sind. Dabei wird bevorzugt eine Verbindung der Formel (III) mit einer Verbindung der Formel (IV) vorzugsweise in einem geeigneten Lösungsmittel (bevorzugt einem Gemisch aus Acetonitril und Wasser) gelöst und gegebenenfalls gerührt (bevorzugt 30 min bei Raumtemperatur). Anschliessend wird eine Verbindung der Formel (V) zugegeben und gegebenenfalls weiter gerührt (bevorzugt 15 h bei Raumtemperatur). Das gegebenenfalls vorhandene Lösungsmittel wird anschliessend bevorzugt im Vakuum entfernt. Die dabei hergestellten Verbindungen können z. B. mittels HPLC gereinigt sowie in die einzelnen Stereoisomere getrennt werden. Gegebenenfalls kann es bevorzugt sein, die Umsetzung in Gegenwart einer Lewis Säure (z. B. Indiumtrichlorid, Bortrifluoridetherat, Trimethylaluminium, Lithiumchlorid, Aluminiumtrichlorid, Scandiumtriflat, Zinkchlorid, Ytterbiumtriflat, Magnesiumtriflat, Magnesiumbromid, Zirkoniumchlorid, Titan(IV)chlorid oder Zinntetrachlorid) oder einer Brønstedt-Säure durchzuführen.

Bei den auf diese Weise erhaltenen Verbindungen wurde gefunden, daß sowohl die Verbindungen der Formel (I) und (II) mit (R)-Konfiguration an der Phenylglycineinheit als auch die entsprechenden (S)-konfigurierten Verbindungen sehr wirksame Faktor Xa-Inhibitoren sind, wobei die (S)-konfigurierten Verbindungen bei gleicher Substitution etwas bessere inhibitorische Eigenschaften besitzen. Bevorzugt werden erfindungsgemäß also Verbindungen der Formel (I) und (II) mit (S)-Konfiguration, wobei auch Verbindungen mit (R)-Konfiguration, sowie Gemische in jedem Mischungsverhältnis, sehr gute inhibitorische Eigenschaften besitzen und ebenfalls Gegenstand dieser Erfindung sind.

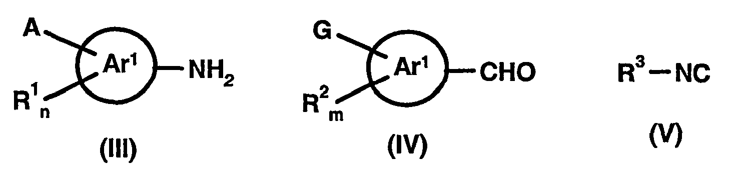

Alternativ können Verbindungen der Formeln (I) oder (II) z. B. analog zu den in WO0230880, WO02057236, WO0112600, WO0071493, WO0071508, WO0071507, WO0035858, WO02068390, W00216312 und WO0190051 beschriebenen Verfahren hergestellt werden.

3-Aminobenzamidin ist kommerziell erhältlich; 3-Amino-4-hydroxybenzamidin kann aus kommerziell erhältlichem 4-Hydroxy-3-nitrobenzonitril über eine Pinner Reaktion (A. Pinner, F. Klein, Ber. 10, 1889 (1877); 11, 4, 1475 (1878); 16, 352, 1643 (1883)) zu 4-Hydroxy-3-nitro-benzamidin und anschliessender Reduktion mit H₂-Pd/C hergestellt werden. Analog können auch weitere Benzamidine (wie z.B. 3-Amino-4-chloro-benzamidin) hergestellt werden.

Glycosylierte Arylverbindungen (z. B. Glycosylierte Benzaldehyde) können z. B. nach den in Kleine et al. Carbohydrate Research 1985, 142, 333-337 und Brewster et al. Tetrahedron Letters 1979, 5051-5054 beschriebenen Verfahren hergestellt werden.

Helicin (Salicylaldehyd-β-D-glucosid) ist kommerziell erhältlich.

Eine Verbindung oder pharmazeutische Zusammensetzung der vorliegenden Erfindung kann zur Hemmung von Faktor Xa-Aktivität, zur Vorbeugung und/oder Behandlung von thromboembolytischen Erkrankungen, arterieller Restenose, Blutvergiftung, Krebs, akuten Entzündungen oder sonstigen Erkrankungen, die durch Faktor Xa-Aktivität vermittelt werden, und insbesondere von venösen Thrombosen, Ödemen oder Entzündungen, von "Deep Vein Thrombosis", Lungenembolien, thromboembolytischen Komplikationen nach größeren Operationen, bei der Gefäßchirurgie, längerer Immobilisierung, Knochenbrüchen der unteren Extremitäten etc., von arteriellen Thrombosen, insbesondere der Herzkranzgefäße bei myokardialem Infarkt sowie Arteriosklerose, Apoplexie, Angina pectoris, Claudicatio intermittens verwendet werden, um nur einige Indikationen zu nennen.

Allgemein sollen, wie eingangs erwähnt wurde, die erfindungsgemäßen Wirkstoffe eine möglichst hohe Inhibierungswirkung gegenüber Faktor Xa bei möglichst hoher Selektivität aufweisen. Die Selektivität wurde im vorliegenden Fall durch Vergleich der Inhibierungswirkung gegenüber Faktor Xa sowie Tryptase, Trypsin, Plasmin, Thrombin und weiteren Serin-Proteinasen bestimmt. Des weiteren sind die vorliegenden/erfindungsgemässen Verbindungen als Inhibitoren weiterer Enzyme der Blutgerinnungskaskade (extrinsisch und intrinsisch) wie z.B. Faktor II, Faktor VII, Faktor VIIa, Faktor IX, Faktor IXa und Faktor X von Interesse.

Wie oben erwähnt, liegt die therapeutische Verwendung der Verbindungen der Formeln (I) oder (II), ihrer pharmakologisch akzeptablen Salze bzw. Solvate und Hydrate sowie Formulierungen und pharmazeutischen Zusammensetzungen im Rahmen der vorliegenden Erfindung.

Auch die Verwendung dieser Wirkstoffe zur Herstellung von Arzneimitteln zur Vorbeugung und/oder Behandlung von thromboembolytischen Erkrankungen ist Gegenstand der vorliegenden Erfindung. Im allgemeinen werden Verbindungen der Formeln (I) oder (II) unter Anwendung der bekannten und akzeptablen Modi, entweder einzeln oder in Kombination mit einem beliebigen anderen therapeutischen Mittel verabreicht. Die Verabreichung kann z.B. auf einem der folgenden Wege erfolgen: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere Wirkstoffe beinhalten, die gewöhnlich zur Vorbeugung und/oder Behandlung von thromboembolytischen Erkrankungen eingesetzt werden wie z.B. Warfarin etc.

Zur Vorbeugung und/oder Behandlung der oben beschriebenen Erkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 0,1 µg bis 10 mg/kg Körpergewicht pro Tag geeignet, wobei eine bevorzugte Dosis 0,1 bis 4 mg/kg pro Tag ist. In geeigneten Fällen kann die Dosis auch unter oder über den oben angegebenen Werten liegen.

Die tägliche Dosis kann beispielsweise in 1, 2, 3 oder 4 Einzeldosen verabreicht werden. Auch ist es möglich, die Dosis z.B. für eine Woche als Einzeldosis zu verabreichen.

Die nachfolgenden Beispiele sollen die Erfindung verdeutlichen. Die Stereochemie von 3,4,5-Trihydroxy-6-hydroxymethyl-tetrahydropyran-2-yloxy entspricht der von β-D-Glucose bzw. bei den Beispielen 58, 59 und 60 der von β-D-Galactose.

### Beispiele

### Allgemeine Arbeitsvorschrift:

1 mmol Amin (II) und 1 mmol Aldehyd (III) werden in 20 ml Acetonitril/Wasser (Mischungsverhältnis von 1:0 bis 1:1) 30 min bei Raumtemperatur gerührt. Anschließend wird 1 mmol Isonitril (IV) zugegeben und weitere 15h gerührt. Das Lösungsmittel wird im Vakuum entfernt und der Rückstand mittels HPLC gereinigt.

### BEISPIEL 1: 2-(3-Carbamimidoyl-phenylamino)-N-(2-trifluoromethyl-benzyl)-2-[2-(3,4,5-trihydroxy-6-hydroxymethyl-tetrahydro-pyran-2-yloxy)-phenyl]-acetamid

C₂₉H₃₁F₃N₄O₇ (604.5882)
ESI-TOF-MS: 605 [M+H]

### BEISPIEL 2: 2-(3-Carbamimidoyl-phenylamino)-N-(2,3-dihydro-benzo[1,4]dioxin-6-yl)-2-[2-(3,4,5-trihydroxy-6-hydroxymethyl-tetrahydro-pyran-2-yloxy)-phenyl]-acetamid

C₂₉H₃₂N₄O₉ (580.5998)
ESI-TOF-MS: 581 [M+H]

### BEISPIEL 3: 2-(3-Carbamimidoyl-phenylamino)-N-[3-(2-oxo-pyrrolidin-1-yl)-propyl]-2-[2-(3,4,5-trihydroxy-6-hydroxymethyl-tetrahydro-pyran-2-yloxy)-phenyl]-acetamid

C₂₈H₃₇N₅O₈ (571.6358)
ESI-TOF-MS: 572 [M+H]

### BEISPIEL 4: 2-(3-Carbamimidoyl-phenylamino)-N-(4-phenoxy-phenyl)-2-[2-(3,4,5-trihydroxy-6-hydroxymethyl-tetrahydro-pyran-2-yloxy)-phenyl]-acetamid

C₃₃H₃₄N₄O₈ (614.6609)
ESI-TOF-MS: 615 [M+H]

### BEISPIEL 5: 2-(3-Carbamimidoyl-phenylamino)-N-(3,3-diphenyl-propyl)-2-[2-(3,4,5-trihydroxy-6-hydroxymethyl-tetrahydro-pyran-2-yloxy)-phenyl]-acetamid

C₃₆H₄₀N₄O₇ (640.7428)
ESI-TOF-MS: 641 [M+H]

### BEISPIEL 6: 2-(3-Carbamimidoyl-phenylamino)-N-(3-phenoxy-phenyl)-2-[2-(3,4,5-trihydroxy-6-hydroxymethyl-tetrahydro-pyran-2-yloxy)-phenyl]-acetamid

C₃₃H₃₄N₄O₈ (614.6609)
ESI-TOF-MS: 615 [M+H]

### BEISPIEL 7: 2-(3-Carbamimidoyl-phenylamino)-N-(4-methoxy-biphenyl-3-yl)-2-[2-(3,4,5-trihydroxy-6-hydroxymethyl-tetrahydro-pyran-2-yloxy)-phenyl]-acetamid

C₃₄H₃₆N₄O₈ (628.6880)
ESI-TOF-MS: 629 [M+H]

### BEISPIEL 8: 2-(3-Carbamimidoyl-phenylamino)-N-(4-morpholin-4-yl-phenyl)-2-[2-(3,4,5-trihydroxy-6-hydroxymethyl-tetrahydro-pyran-2-yloxy)-phenyl]-acetamid

C₃₁H₃₇N₅O₈ (607.6692)
ESI-TOF-MS: 608 [M+H]

### BEISPIEL 9: 2-(3-Carbamimidoyl-phenylamino)-N-(4-benzoyl-phenyl)-2-[2-(3,4,5-trihydroxy-6-hydroxymethyl-tetrahydro-pyran-2-yloxy)-phenyl]-acetamid

C₃₄H₃₄N₄O₈ (626.6721)
ESI-TOF-MS: 627 [M+H]

### BEISPIEL 10: 2-(3-Carbamimidoyl-phenylamino)-N-(3-benzoyl-phenyl)-2-[2-(3,4,5-trihydroxy-6-hydroxymethyl-tetrahydro-pyran-2-yloxy)-phenyl]-acetamid

C₃₄H₃₄N₄O₈ (626.6721)
ESI-TOF-MS: 627 [M+H]

### BEISPIEL 11: 2-(3-Carbamimidoyl-phenylamino)-N-(4-tert-butyl-benzyl)-2-[2-(3,4,5-trihydroxy-6-hydroxymethyl-tetrahydro-pyran-2-yloxy)-phenyl]-acetamid

C₃₂H₄₀N₄O₇ (592.6982)
ESI-TOF-MS: 593 [M+H]

### BEISPIEL 12: 2-(2-Hydroxy-5-carbamimidoyl-phenylamino)-N-(4-morpholin-4-yl-phenyl)-2-[2-(3,4,5-trihydroxy-6-hydroxymethyl-tetrahydro-pyran-2-yloxy)-phenyl]-acetamid

C₃₁H₃₇N₅O₉ (623.6686)
ESI-TOF-MS: 624 [M+H]

### BEISPIEL 13: 2-(3-Carbamimidoyl-phenylamino)-N-(3-methoxy-benzyl)-2-[2-(3,4,5-trihydroxy-6-hydroxymethyl-tetrahydro-pyran-2-yloxy)-phenyl]-acetamid

C₂₉H₃₄N₄O₈ (566.6163)
ESI-TOF-MS: 567 [M+H]

### BEISPIEL 14: N-(4-Acetyl-phenyl)-2-(3-carbamimidoyl-phenylamino)-2-[2-(3,4,5-trihydroxy-6-hydroxymethyl-tetrahydro-pyran-2-yloxy)-phenyl]-acetamid

C₂₉H₃₂N₄O₈ (564.6004)
ESI-TOF-MS: 565 [M+H]

### BEISPIEL 15: 2-(3-Carbamimidoyl-phenylamino)-N-(3-trifluoromethyl-benzyl)-2-[2-(3,4,5-trihydroxy-6-hydroxymethyl-tetrahydro-pyran-2-yloxy)-phenyl]-acetamid

C₂₉H₃₁F₃N₄O₇ (604.5882)
ESI-TOF-MS: 605 [M+H]

### BEISPIEL 16: 2-(3-Carbamimidoyl-phenylamino)-N-(2-cyclohex-1-enyl-ethyl)-2-[2-(3,4,5-trihydroxy-6-hydroxymethyl-tetrahydro-pyran-2-yloxy)-phenyl]-acetamid

C₂₉H₃₈N₄O₇ (554.6488)
ESI-TOF-MS: 555 [M+H]

### BEISPIEL 17: 2-(3-Carbamimidoyl-phenylamino)-N-[2-(3,4-dimethoxy-phenyl)-ethyl]-2-[2-(3,4,5-trihydroxy-6-hydroxymethyl-tetrahydro-pyran-2-yloxy)-phenyl]-acetamid

C₃₁H₃₈N₄O₉ (610. 6699)
ESI-TOF-MS: 611 [M+H]

### BEISPIEL 18: 2-(3-Carbamimidoyl-phenylamino)-N-(3-morpholin-4-yl-propyl)-2-[2-(3,4,5-trihydroxy-6-hydroxymethyl-tetrahydro-pyran-2-yloxy)-phenyl]-acetamid

C₂₈H₃₉N₅O₈ (573.6517)
ESI-TOF-MS: 574 [M+H]

### BEISPIEL 19: 2-(3-Carbamimidoyl-phenylamino)-N-(4-trifluoromethyl-benzyl)-2-[2-(3,4,5-trihydroxy-6-hydroxymethyl-tetrahydro-pyran-2-yloxy)-phenyl]-acetamid

C₂₉H₃₁F₃N₄O₇ (604.5882)
ESI-TOF-MS: 605 [M+H]

### BEISPIEL 20: N-[1-(4-Bromo-phenyl)-ethyl]-2-(3-carbamimidoyl-phenylamino)-2-[2-(3,4,5-trihydroxy-6-hydroxymethyl-tetrahydro-pyran-2-yloxy)-phenyl]-acetamid

C₂₉H₃₃BrN₄O₇ (629.5130)
ESI-TOF-MS: 630 [M+H]

### BEISPIEL 21: N-Benzo[1,3]dioxol-5-ylmethyl-2-(3-carbamimidoyl-phenylamino)-2-[2-(3,4,5-trihydroxy-6-hydroxymethyl-tetrahydro-pyran-2-yloxy)-phenyl]-acetamid

C₂₉H₃₂N₄O₉ (580.5998)
ESI-TOF-MS: 581 [M+H]

### BEISPIEL 22: 2-(3-Carbamimidoyl-phenylamino)-N-(3-phenyl-propyl)-2-[2-(3,4,5-trihydroxy-6-hydroxymethyl-tetrahydro-pyran-2-yloxy)-phenyl]-acetamid

C₃₀H₃₆N₄O₇ (564.6440)
ESI-TOF-MS: 565 [M+H]

### BEISPIEL 23: 2-(3-Carbamimidoyl-phenylamino)-N-(3,5-dimethyl-benzyl)-2-[2-(3,4,5-trihydroxy-6-hydroxymethyl-tetrahydro-pyran-2-yloxy)-phenyl]-acetamid

C₃₀H₃₆N₄O₇ (564.6440)
ESI-TOF-MS: 565 [M+H]

### BEISPIEL 24: 2-(3-Carbamimidoyl-phenylamino)-N-(3-cyano-phenyl)-2-[2-(3,4,5-trihydroxy-6-hydroxymethyl-tetrahydro-pyran-2-yloxy)-phenyl]-acetamid

C₂₈H₂₉N₅O₇ (547.5726)
ESI-TOF-MS: 548 [M+H]

### BEISPIEL 25: 2-(3-Carbamimidoyl-phenylamino)-N-(3,4-dichloro-benzyl)-2-[2-(3,4,5-trihydroxy-6-hydroxymethyl-tetrahydro-pyran-2-yloxy)-phenyl]-acetamid

C₂₈H₃₀C₁₂N₄O₇ (605.4799)
ESI-TOF-MS: 606 [M+H]

### BEISPIEL 26: N-(3-Acetyl-phenyl)-2-(3-carbamimidoyl-phenylamino)-2-[2-(3,4,5-trihydroxy-6-hydroxymethyl-tetrahydro-pyran-2-yloxy)-phenyl]-acetamid

C₂₉H₃₂N₄O₈ (564.6004)
ESI-TOF-MS: 565 [M+H]

### BEISPIEL 27: 2-(3-Carbamimidoyl-phenylamino)-2-[2-(3,4,5-trihydroxy-6-hydroxymethyl-tetrahydro-pyran-2-yloxy)-phenyl]-N-(1,2,2-trimethyl-propyl)-acetamid

C₂₇H₃₈N₄O₇ (530.6265)
ESI-TOF-MS: 531 [M+H]

### BEISPIEL 28: N-Allyl-2-(3-carbamimidoyl-phenylamino)-2-[2-(3,4,5-trihydroxy-6-hydroxymethyl-tetrahydro-pyran-2-yloxy)-phenyl]-acetamid

C₂₄H₃₀N₄O₇ (486.5293)
ESI-TOF-MS: 487 [M+H]

### BEISPIEL 29: N-(3-Butoxy-propyl)-2-(3-carbamimidoyl-phenylamino)-2-[2-(3,4,5-trihydroxy-6-hydroxymethyl-tetrahydro-pyran-2-yloxy)-phenyl]-acetamid

C₂₈H₄₀N₄O₈ (560.6530)
ESI-TOF-MS: 561 [M+H]

### BEISPIEL 30: 2-(3-Carbamimidoyl-phenylamino)-N-(3,7-dimethyl-octa-2,6-dienyl)-2-[2-(3,4,5-trihydroxy-6-hydroxymethyl-tetrahydro-pyran-2-yloxy)-phenyl]-acetamid

C₃₁H₄₂N₄O₇ (582.7030)
ESI-TOF-MS: 583 [M+H]

### BEISPIEL 31: 2-(3-Carbamimidoyl-phenylamino)-N-furan-2-ylmethyl-2-[2-(3,4,5-trihydroxy-6-hydroxymethyl-tetrahydropyran-2-yloxy)-phenyl]-acetamid

C₂₆H₃₀N₄O₈ (526.5510)
ESI-TOF-MS: 527 [M+H]

### BEISPIEL 32: 2-(3-Carbamimidoyl-phenylamino)-N-(3-isopropoxy-propyl)-2,[2-(3,4,5-trihydroxy-6-hydroxymethyl-tetrahydro-pyran-2-yloxy)-phenyl]-acetamid

C₂₇H₃₈N₄O₈ (546.6259)
ESI-TOF-MS: 547 [M+H]

### BEISPIEL 33: 3-{2-(3-Carbamimidoyl-phenylamino)-2-[2-(3,4,5-trihydroxy-6-hydroxymethyl-tetrahydro-pyran-2-yloxy)-phenyl]-acetylamino}-propionsäure ethylester

C₂₆H₃₄N₄O₉ (546.5823)
ESI-TOF-MS: 547 [M+H]

### BEISPIEL 34: N-tert-Butyl-2-(3-carbamimidoyl-phenylamino)-2-[2-(3,4,5-trihydroxy-6-hydroxymethyl-tetrahydro-pyran-2-yloxy)-phenyl]-acetamid

C₂₅H₃₄N₄O₇ (502.5723)
ESI-TOF-MS: 503 [M+H]

### BEISPIEL 35: 2-(3-Carbamimidoyl-phenylamino)-N-pyridin-4-ylmethyl-2-[2-(3,4,5-trihydroxy-6-hydroxymethyl-tetrahydropyran-2-yloxy)-phenyl]-acetamid

C₂₇H₃₁N₅O₇ (537.5774)
ESI-TOF-MS: 538 [M+H]

### BEISPIEL 36: 2-(3-Carbamimidoyl-phenylamino)-N-methyl-2-[2-(3,4,5-trihydroxy-6-hydroxymethyl-tetrahydro-pyran-2-yloxy)-phenyl]-acetamid

C₂₂H₂₈N₄O₇ (460.4911)
ESI-TOF-MS: 461 [M+H]

### BEISPIEL 37: 2-(3-Carbamimidoyl-phenylamino)-N-(1,3-dimethyl-butyl)-2-[2-(3,4,5-trihydroxy-6-hydroxymethyl-tetrahydro-pyran-2-yloxy)-phenyl]-acetamid

C₂₇H₃₈N₄O₇ (530.6265)
ESI-TOF-MS: 531 [M+H]

### BEISPIEL 38: N-(4-Benzoyl-phenyl)-2-(3-carbamimidoyl-phenylamino)-2-[2-(3,4,5-trihydroxy-6-hydroxymethyl-tetrahydro-pyran-2-yloxy)-phenyl]-acetamid

C₃₄H₃₄N₄O₈ (626.6721)
ESI-TOF-MS: 627 [M+H]

### BEISPIEL 39: 2-(5-Carbamimidoyl-2-hydroxy-phenylamino)-N-(4-morpholin-4-yl-phenyl)-2-[2-(3,4,5-trihydroxy-6-hydroxy-methyl-tetrahydro-pyran-2-yloxy)-phenyl]-acetamid

C₃₁H₃₇N₅O₉ (623.6686)
ESI-TOF-MS: 624 [M+H]

### BEISPIEL 40: 2-(3-Carbamimidoyl-phenylamino)-N-(2,6-dimethyl-phenyl)-2-[2-(3,4,5-trihydroxy-6-hydroxymethyl-tetrahydro-pyran-2-yloxy)-phenyl]-acetamid

C₂₉H₃₄N₄O₇ (550.6169)
ESI-TOF-MS: 551 [M+H]

### BEISPIEL 41: 2-(3-Carbamimidoyl-phenylamino)-N-(4-nitro-phenyl)-2-[2-(3,4,5-trihydroxy-6-hydroxymethyl-tetrahydro-pyran-2-yloxy)-phenyl]-acetamid

C27H29N5O9 (567.5603)
ESI-TOF-MS: 568 [M+H]

### BEISPIEL 42: N-(4-Amino-phenyl)-2-(3-carbamimidoyl-phenylamino)-2-[2-(3,4,5-trihydroxy-6-hydroxymethyl-tetrahydro-pyran-2-yloxy)-phenyl]-acetamid

C27H31N5O7 (537.5774)
ESI-TOF-MS: 538 [M+H]

### BEISPIEL 43: 2-{2-(3-Carbamimidoyl-phenylamino)-2-[2-(3,4,5-trihydroxy-6-hydroxymethyl-tetrahydro-pyran-2-yloxy)-phenyl]-acetylamino}-benzoesäure methyl ester

C₂₉H₃₂N₄O₉ (580.5998)
ESI-TOF-MS: 581 [M+H]

### BEISPIEL 44: 2-(5-Carbamimidoyl-2-chloro-phenylamino)-N-(4-morpholin-4-yl-phenyl)-2-[2-(3,4,5-trihydroxy-6-hydroxy-methyl-tetrahydro-pyran-2-yloxy)-phenyl]-acetamid

C₃₁H₃₆ClN₅O₈ (642.1143)
ESI-TOF-MS: 643 [M+H]

### BEISPIEL 45: 2-(3-Carbamimidoyl-phenylamino)-N-[4-(morpholin-4-carbonyl)-phenyl]-2-[2-(3,4,5-trihydroxy-6-hydroxymethyl-tetrahydro-pyran-2-yloxy)-phenyl]-acetamid

C₃₂H₃₇N₅O₉ (635.6798)
ESI-TOF-MS: 636 [M+H]

### BEISPIEL 46: 2-(5-Carbamimidoyl-2-methylamino-phenylamino)-N-(4-morpholin-4-yl-phenyl)-2-[2-(3,4,5-trihydroxy-6-hydroxymethyl-tetrahydro-pyran-2-yloxy)-phenyl]-acetamid

C₃₂H₄₀N₆O₈ (636.7110)
ESI-TOF-MS: 637 [M+H]

### BEISPIEL 47: N-(2-Bromo-phenyl)-2-(3-carbamimidoyl-phenyl-amino)-2-[2-(3,4,5-trihydroxy-6-hydroxymethyl-tetrahydro-pyran-2-yloxy)-phenyl]-acetamid

C₂₇H₂₉BrN₄O₇ (601.4588)
ESI-TOF-MS: 602 [M+H]

### BEISPIEL 48: 2-{2-(3-Carbamimidoyl-phenylamino)-2-[2-(3,4,5-trihydroxy-6-hydroxymethyl-tetrahydro-pyran-2-yloxy)-phenyl]-acetylamino}-benzoesäure

C₂₈H₃₀N₄O₉ (566.5727)
ESI-TOF-MS: 567 [M+H]

### BEISPIEL 49: 2-(3-Carbamimidoyl-phenylamino)-N-quinolin-6-yl-2-[2-(3,4,5-trihydroxy-6-hydroxymethyl-tetrahydro-pyran-2-yloxy)-phenyl]-acetamid

C₃₀H₃₁N₅O₇ (573.6109)
ESI-TOF-MS: 574 [M+H]

### BEISPIEL 50: 2-{2-{3-[N-(3-Fluoro-benzyl)-carbamimidoyl]-phenylamino}-2-[2-(3,4,5-trihydroxy-6-hydroxymethyl-tetrahydro-pyran-2-yloxy)-phenyl]-acetylamino}-benzoesäure methyl ester

C₃₆H₃₇FN₄O₉ (688.7161)
ESI-TOF-MS: 689 [M+H]

### BEISPIEL 51: N-(2-Benzoyl-4-chloro-phenyl)-2-(3-carbamimidoyl-phenylamino)-2-[2-(3,4,5-trihydroxy-6-hydroxymethyl-tetrahydro-pyran-2-yloxy)-phenyl]-acetamid

C₃₄H₃₃ClN₄O₈ (661.1171)
ESI-TOF-MS: 662 [M+H]

### BEISPIEL 52: 2-(3-Carbamimidoyl-phenylamino)-N-[3-chloro-4-(morpholin-4-carbonyl)-phenyl]-2-[2-(3,4,5-trihydroxy-6-hydroxymethyl-tetrahydro-pyran-2-yloxy)-phenyl]-acetamid

C₃₂H₃₆ClN₅O₉ (670.1248)
ESI-TOF-MS: 671 [M+H]

### BEISPIEL 53: 2-(3-Carbamimidoyl-phenylamino)-N-[2-methyl-4-(morpholin-4-carbonyl)-phenyl]-2-[2-(3,4,5-trihydroxy-6-hydroxymethyl-tetrahydro-pyran-2-yloxy)-phenyl]-acetamid

C₃₃H₃₉N₅O₉ (649.7069)
ESI-TOF-MS: 650 [M+H]

### BEISPIEL 54: 2-(5-Carbamimidoyl-2-hydroxy-phenylamino)-N-[4-(morpholin-4-carbonyl)-phenyl]-2-[2-(3,4,5-trihydroxy-6-hydroxymethyl-tetrahydro-pyran-2-yloxy)-phenyl]-acetamid

C₃₂H₃₇N₅O₁₀ (651.6792)
ESI-TOF-MS: 652 [M+H]

### BEISPIEL 55: 2-(3-Carbamimidoyl-phenylamino)-N-(3,4-difluoro-phenyl)-2-[2-(3,4,5-trihydroxy-6-hydroxymethyl-tetrahydro-pyran-2-yloxy)-phenyl]-acetamid

C₂₇H₂₈F₂N₄O₇ (558.5436)
ESI-TOF-MS: 559 [M+H]

### BEISPIEL 56: 4-{2-(3-Carbamimidoyl-phenylamino)-2-[2-(3,4,5-trihydroxy-6-hydroxymethyl-tetrahydro-pyran-2-yloxy)-phenyl]-acetylamino}-benzoesäure methyl ester

C₂₉H₃₂N₄O₉ (580.5998)
ESI-TOF-MS: 581 [M+H]

### BEISPIEL 57: 2-(3-Carbamimidoyl-phenylamino)-N-(3,4-dimethoxy-phenyl)-2-[2-(3,4,5-trihydroxy-6-hydroxymethyl-tetrahydro-pyran-2-yloxy)-phenyl]-acetamid

C₂₉H₃₄N₄O₉ (582.6157)
ESI-TOF-MS: 583 [M+H]

### BEISPIEL 58: Essigsäure 3,4,5-triacetoxy-6-{2-[(5-carbamimidoyl-2-hydroxy-phenylamino)-(4-morpholin-4-yl-phenylcarbamoyl)-methyl]-phenoxy}-tetrahydro-pyran-2-ylmethyl ester

C₃₉H₄₅N₅O₁₃ (791.8192)
ESI-TOF-MS: 792 [M+H]

### BEISPIEL 59: 2-(3-Carbamimidoyl-phenylamino)-N-(4-morpholin-4-yl-phenyl)-2-[2-(3,4,5-trihydroxy-6-hydroxy-methyl-tetrahydro-pyran-2-yloxy)-phenyl]-acetamid

C₃₁H₃₇N₅O₈ (607.6692)
ESI-TOF-MS: 608 [M+H]

### BEISPIEL 60: 2-(5-Carbamimidoyl-2-hydroxy-phenylamino)-N-(4-morpholin-4-yl-phenyl)-2-[2-(3,4,5-trihydroxy-6-hydroxymethyl-tetrahydro-pyran-2-yloxy)-phenyl]-acetamid

C₃₁H₃₇N₅O₉ (623.6686)
ESI-TOF-MS: 624 [M+H]

### BEISPIEL 61: 2-(3-Carbamimidoyl-phenylamino)-N-(4-methoxy-phenyl)-2-[2-(3,4,5-trihydroxy-6-hydroxymethyl-tetrahydro-pyran-2-yloxy)-phenyl]-acetamid

C₂₈H₃₂N₄O₈ (552.5892)
ESI-TOF-MS: 553 [M+H]

### BEISPIEL 62: 2-(3-Carbamimidoyl-phenylamino)-2-[3-ethoxy-2-(3,4,5-trihydroxy-6-hydroxymethyl-tetrahydro-pyran-2-yloxy)-phenyl]-N-[4-(morpholine-4-carbonyl)-phenyl]-acetamid

C₃₄H₄₁N₅O₁₀ (679.7334)
ESI-TOF-MS: 680 [M+H]

### BEISPIEL 63: 2-(3-Carbamimidoyl-phenylamino)-2-[3-ethoxy-2-(3,4,5-trihydroxy-6-hydroxymethyl-tetrahydro-pyran-2-yloxy)-phenyl]-N-(4-morpholin-4-yl-phenyl)-acetamid

C₃₃H₄₁N₅O₉ (651.7228)
ESI-TOF-MS: 652 [M+H]

Um die Inhibierungswirkung gegenüber der Faktor Xa-Aktivität zu zeigen, wurden chromogene Peptid-Substrate verwendet. Die Hemmung der amidolytischen Aktivität von Faktor Xa durch die oben beschriebenen Verbindungen wurde wie folgt gezeigt. Die Messungen wurden bei Raumtemperatur in Mikrotiterplatten durchgeführt. Die Verbindungen wurden in Dimethylsulfoxid aufgelöst und 5 µl dieser Lösung (1mM, 100µM, 10µM, 1µM) wurden zu 35µl einer 2.15 nM Lösung von humanem rekombinantem Faktor Xa (Enzyme Research Laboratories, South Bend, IN, USA) in einem Puffer (pH: 8,0 und unter Verwendung von 50 mM Tris-HCl, 100 mM NaCl, 0,1 % PEG 6000 und 0,05 % Tween 80) gegeben. Schließlich wurden 10µl einer 25µM Lösung MeSO₂-D-CHA-Gly-Arg-AMC Acetat ("Spectrozym fXa", American Diagnostica, Pfungstadt, Germany) in Puffer zugesetzt und die Hydrolyse des Substrats mit einem Spektralphotometer Spectra Fluor Plus (Tecan, Crailsheim, Deutschland) bei folgenden Wellenlängen: Excitation: 360 nm, Emission 465 nm über einen Zeitraum von 20 min verfolgt. Die Berechnung der IC₅₀-Werte erfolgte mit Hilfe des Programms "GraFit 4" der Firma Erithacus Software Ltd. (Staines, Middlesex, UK). Unter der Annahme, dass die Kinetik eine kompetitive Inhibition aufweist, konnte der Kᵢ-Wert nach der Cheng-Prusoff-Gleichung: Kᵢ = IC₅₀/(1+ [S]/Kₘ]) bestimmt werden (Cheng and Prusoff, Biochemical Pharmacology 1973, 22: 3099-3108). Das selbe Verfahren aber unter Verwendung von Tosyl-glycyl-prolyl-lysin-4-nitranilidacetat als Substrat in Hepes-Puffer (pH 7.8), wurde zur Bestimmung der Hemmung der proteolytischen Aktivität von rekombinanter humaner Tryptase (Promega, Madison, WI, USA) durch die besagten Verbindungen verwendet.

Die IC₅₀ Werte der oben genannten Beispiele liegen im Bereich von 0.1 nM bis 1µM.

## Patentansprüche

1. Verbindungen der Formel (I): 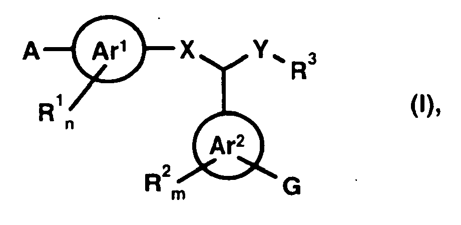 worin
A ein Wasserstoff oder eine Gruppe der Formel -C(=NR⁴)NH₂ ist, wobei R⁴ ein Wasserstoff oder eine Heteroaralkylgruppe ist,
Ar¹ eine Arylgruppe mit 6 bis 10 Ringatomen oder eine Heteroarylgruppe mit 5, 6, 7, 8, 9 oder 10 Ringatomen ist;
Ar² eine Arylgruppe mit 6 bis 10 Ringatomen oder eine Heteroarylgruppe mit 5, 6, 7, 8, 9 oder 10 Ringatomen ist,
die Reste R¹ unabhängig voneinander eine Hydroxy-, eine C₁-C₄-Alkyloxy-, eine C₁-C₄-Alkylaminogruppe oder ein Halogenatom sind;
die Reste R² unabhängig voneinander eine Hydroxy- oder eine C₁-C₄-Alkyloxygruppe sind;
R³ eine Gruppe der Formel -U-V-W ist, wobei U eine gegebenenfalls substituierte C₆-C₁₀-Arylgruppe oder eine gegebenenfalls substituierte Heteroarylgruppe mit 5 bis 10 Ringatomen und 1, 2, 3 oder 4 Heteroatomen, die aus O, S und N ausgewählt sind, ist; V eine direkte Bindung, ein Sauerstoffatom, ein Schwefelatom, eine Gruppe der Formel NR¹¹, wobei R¹¹ ein Wasserstoffatom, eine C₁-C₄-Alkyl-, eine C₁-C₄-Heteroalkyl-, eine C₇-C₁₂-Aralkylgruppe oder eine C₆-C₁₂-Heteroaralkylgruppe ist, CO, SO, SO₂ oder SO₂NH ist und W ein Wasserstoffatom, ein C₁-C₄-Alkyl-, C₂-C₄-Alkenyl-, C₂-C₄-Alkinyl-, C₁-C₄-Heteroalkyl-, Aryl-, Heteroaryl-, Cycloalkyl-, Alkylcycloalkyl-, Aralkyl-, Heteroalkylcycloalkyl-, Heterocycloalkyl- oder ein Heteroaralkylrest ist;
G eine Glycosyloxygruppe ist;
X eine Gruppe der Formel NR⁵ ist, wobei R⁵ ein Wasserstoffatom oder eine C₁-C₄-Alkylgruppe ist;
Y eine Gruppe der Formel CONR⁶ ist, wobei R⁶ ein Wasserstoffatom, eine C₁-C₄-Alkyl-, eine C₁-C₄-Heteroalkyl-, oder eine C₇-C₁₂-Aralkylgruppe ist;
n gleich 0 oder 1 ist und
m gleich 0 oder 1 ist
oder ein pharmakologisch akzeptables Salz, Solvat, Hydrat oder eine pharmakologisch akzeptable Formulierung derselben.

2. Verbindungen nach Anspruch 1, wobei A eine Gruppe der Formel -C(=NH)NH₂ ist.

3. Verbindungen nach Anspruch 1 oder 2, wobei Ar¹ eine Phenyl- oder eine Heteroarylgruppe mit 5, 6, 7, 8, 9 oder 10 Ringatomen und 1, 2, 3 oder 4 Ring-Heteroatomen, die aus O, S und N ausgewählt sind ist.

4. Verbindungen nach Anspruch 1 oder 2, wobei Ar¹ eine Phenylgruppe ist, an die die Gruppen A und X in meta-Position zueinander gebunden sind.

5. Verbindungen nach einem der Ansprüche 1, 2, 3 oder 4, wobei Ar² eine Phenylgruppe ist.

6. Verbindungen nach einem der Ansprüche 1, 2, 3, 4 oder 5, wobei X eine NH-Gruppe ist.

7. Verbindungen nach einem der Ansprüche 1, 2, 3, 4, 5 oder 6, wobei R¹ eine Hydroxygruppe ist.

8. Verbindungen nach einem der Ansprüche 1, 2 , 3, 4, 5 , 6 oder 7, wobei Y eine Gruppe der Formel CONH ist.

9. Verbindungen nach Anspruch 1, wobei U eine gegebenenfalls substituierte Phenylgruppe ist.

10. Verbindungen nach Anspruch 1 oder 9, wobei V eine direkte Bindung oder eine Carbonylgruppe ist.

11. Verbindungen nach Anspruch 1, 9 oder 10, wobei W eine C₁-C₄ Alkylgruppe, eine C₁-C₄ Heteroalkylgruppe, eine gegebenenfalls substituierte Phenylgruppe, eine gegebenenfalls substituierte C₃-C₇ Cycloalkylgruppe, eine gegebenenfalls substituierte Heterocycloalkylgruppe mit 3-7 Ringatomen und 1, 2 oder 3 Heteroatomen (ausgewählt aus O, S und N) oder eine gegebenenfalls substituierte Heteroarylgruppe mit 5 oder 6 Ringatomen und 1, 2, 3 oder 4 Heteroatomen, die aus O, S und N ausgewählt sind, ist.

12. Pharmazeutische Zusammensetzungen, die eine Verbindung nach den Ansprüchen 1, 2, 3, 4, 5, 6, 7, 8, 9, 10 oder 11, und fakultativ Trägerstoffe und/oder Adjuvanzien enthalten.

13. Verwendung einer Verbindung oder einer pharmazeutischen Zusammensetzung nach einem der. Ansprüche 1, 2, 3, 4, 5, 6, 7, 8, 9, 10, 11 oder 12 zur Herstellung eines Medikaments zur Hemmung von Faktor Xa.

14. Verwendung einer Verbindung oder einer pharmazeutischen Zusammensetzung nach einem der Ansprüche 1, 2, 3, 4, 5, 6, 7, 8, 9, 10, 11 oder 12 zur Herstellung eines Medikaments zur Behandlung und/oder Vorbeugung von thromboembolytischen Erkrankungen, arterieller Restenose, Blutvergiftung, Krebs, akuten Entzündungen, oder sonstigen Erkrankungen, die durch Faktor Xa-Aktivität vermittelt werden.

15. Verwendung einer Verbindung oder einer pharmazeutischen Zusammensetzung nach einem der Ansprüche 1, 2, 3, 4, 5, 6, 7, 8, 9, 10, 11 oder 12 zur Herstellung eines Medikaments zum Einsatz in der Gefäßchirurgie.

## Claims

1. Compounds of formula (I): 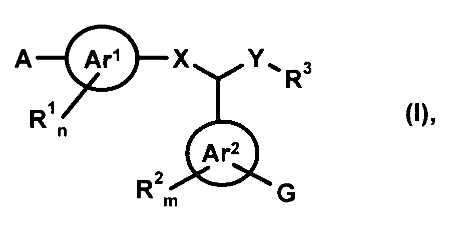 wherein
A is a hydrogen atom or a group of formula -C(=NR⁴)NH₂, wherein R⁴ is a hydrogen atom or a heteroaralkyl,
Ar¹ is an aryl group containing from 6 to 10 ring atoms or a heteroaryl group containing 5, 6, 7, 8, 9 or 10 ring atoms;
Ar² is an aryl group containing from 6 to 10 ring atoms or a heteroaryl group containing 5, 6, 7, 8, 9 or 10 ring atoms;
the radicals R¹ are, each independently of any other(s), a hydroxy group, a C₁-C₄alkyloxy group, a C₁-C₄alkylamino group or a halogen atom;
the radicals R², each independently of any other(s), are a hydroxy group or a C₁-C₄alkyloxy group;
R³ is a group of formula -U-V-W, wherein U is an optionally substituted C₆-C₁₀aryl group or an optionally substituted heteroaryl group containing from 5 to 10 ring atoms and 1, 2, 3 or 4 hetero atoms selected from O, S and N; V is a direct bond, an oxygen atom, a sulphur atom, a group of formula NR¹¹, R¹¹ being a hydrogen atom, a C₁-C₄alkyl, C₁-C₄heteroalkyl, C₇-C₁₂aralkyl or C₆-C₁₂heteroaralkyl group, CO, SO, SO₂ or SO₂NH, and W is a hydrogen atom, an C₁-C₄alkyl, C₂-C₄alkenyl, C₂-C₄alkynyl, C₁-C₄heteroalkyl, aryl, heteroaryl, cycloalkyl, alkylcycloalkyl, aralkyl, heteroalkylcycloalkyl, heterocycloalkyl or heteroaralkyl radical.
G is a glycosyloxy group;
X is a group of formula NR⁵, wherein R⁵ is a hydrogen atom or a C₁-C₄alkyl group;
Y is a group of formula CONR⁶, wherein R⁶ is a hydrogen atom or a C₁-C₄alkyl, C₁-C₄heteroalkyl or C₇-C₁₂aralkyl group;
n is 0 or 1, and
m is 0 or 1,
or a pharmacologically acceptable salt, solvate, hydrate or pharmacologically acceptable formulation thereof.

2. Compounds according to claim 1, wherein A is a group of formula -C(=NH)NH₂.

3. Compounds according to claim 1 or 2, wherein Ar¹ is a phenyl or heteroaryl group having 5, 6, 7, 8, 9 or 10 ring atoms and 1, 2, 3 or 4 ring hetero atoms selected from O, S and N.

4. Compounds according to claim 1 or 2, wherein Ar¹ is a phenyl group to which the groups A and X are bonded in positions meta to one another.

5. Compounds according to one of claims 1, 2, 3 or 4, wherein Ar² is a phenyl group.

6. Compounds according to one of claims 1, 2, 3, 4 or 5, wherein X is an NH group.

7. Compounds according to one of claims 1, 2, 3, 4, 5, or 6, wherein R¹ is a hydroxy group.

8. Compounds according to one of claims 1, 2, 3, 4, 5, 6 or 7, wherein Y is a group of formula CONH.

9. Compounds according to claim 1, wherein U is an optionally substituted phenyl group.

10. Compounds according to claim 1 or 9, wherein V is a direct bond or a carbonyl group.

11. Compounds according to claim 1, 9 or 10, wherein W is a C₁-C₄alkyl group, a C₁-C₄heteroalkyl group, an optionally substituted phenyl group, an optionally substituted C₃-C₇cycloalkyl group, an optionally substituted heterocycloalkyl group having 3-7 ring atoms and 1, 2 or 3 hetero atoms (selected from O, S and N) or an optionally substituted heteroaryl group having 5 or 6 ring atoms and 1, 2, 3 or 4 hetero atoms selected from O, S and N.

12. Pharmaceutical compositions comprising a compound according to claims 1, 2, 3, 4, 5, 6, 7, 8, 9, 10 or 11 and, optionally, carrier substances and/or adjuvants.

13. Use of a compound or of a pharmaceutical composition according to one of claims 1, 2, 3, 4, 5, 6, 7, 8, 9, 10, 11 or 12 for the manufacture of a medicament for inhibiting factor Xa.

14. Use of a compound or of a pharmaceutical composition according to one of claims 1, 2, 3, 4, 5, 6, 7, 8, 9, 10, 11 or 12 for the manufacture of a medicament for the treatment and/or prevention of thromboembolic conditions, arterial restenosis, septicaemia, cancer, acute inflammation or other conditions mediated by factor Xa activity.

15. Use of a compound or of a pharmaceutical composition according to one of claims 1, 2, 3, 4, 5, 6, 7, 8, 9, 10, 11 or 12 for the manufacture of a medicament for utilisation in vascular surgery.

## Revendications

1. Composés de la formule (I) : 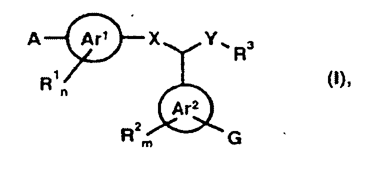 dans laquelle
A est un hydrogène ou un groupe de la formule -C(=NR⁴)NH₂, dans laquelle R⁴ est un hydrogène ou un groupe hétéroaralkyle,
Ar¹ est un groupe aryle avec 6 à 10 atomes dans le cycle ou un groupe hétéroaryle avec 5, 6, 7, 8, 9 ou 10 atomes dans le cycle,
Ar² est un groupe aryle avec 6 à 10 atomes dans le cycle ou un groupe hétéroaryle avec 5, 6, 7, 8, 9 ou 10 atomes dans le cycle,
les radicaux R¹, indépendamment les uns des autres, sont un groupe hydroxyle, alcoxyle en C₁ à C₄, alkylamine en C₁ à C₄ ou un atome d'halogène;
les radicaux R², indépendamment les uns des autres, sont un groupe hydroxyle ou un groupe alcoxyle en C₁ à C₄;
R³ est un groupe de la formule -U-V-W, dans laquelle U est un groupe aryle en C₆ à C₁₀, substitué le cas échéant, ou groupe hétéroaryle avec 5 à 10 atomes dans le cycle, substitué le cas échéant, et 1, 2, 3 ou 4 hétéroatomes, qui sont choisis parmi O, S et N ; V est une liaison directe, un atome d'oxygène, un atome de soufre, un groupe de la formule NR¹¹, dans laquelle R¹¹ est un atome d'hydrogène, un alkyle en C₁ à C₄, un hétéroalkyle en C₁ à C₄, un groupe aralkyle en C₇ à C₁₂ ou un groupe hétéroaralkyle en C₆ à C₁₂, CO, SO, SO₂ ou SO₂NH, et W est un atome d'hydrogène, un groupe alkyle en C₁ à C₄, un groupe alcényle en C₂ à C₄, un groupe alcynyle en C₂ à C₄, un groupe hétéroalkyle en C₁ à C₄, un groupe aryle, un groupe hétéroaryle, un groupe cycloalkyle, un groupe alkylcycloalkyle, un groupe aralkyle, un groupe hétéroalkylcycloalkyle, un groupe hétérocycloalkyle, ou un groupe hétéroaralkyle ;
G est un groupe glycosyloxyle ;
X est un groupe de la formule NR⁵, dans laquelle R⁵ est un atome d'hydrogène ou un groupe alkyle en C₁ à C₄ ;
Y est un groupe de la formule CONR⁶, dans laquelle R⁶ est un atome d'hydrogène, un groupe alkyle en C₁ à C₄, un groupe hétéroalkyle en C₁ à C₄ ou un groupe aralkyle en C₇ à C₁₂ ;
n est égal à 0 ou 1, et
m est égal à 0 ou 1,
ou un sel, un solvate, un hydrate pharmacologiquement acceptables ou une formulation pharmacologiquement acceptable de ces derniers.

2. Composés selon la revendication 1, dans lesquels A est un groupe de la formule -C(=NH)NH₂.

3. Composés selon la revendication 1 ou 2, dans lesquels Ar¹ est un groupe phényle ou hétéroaryle avec 5, 6, 7, 8, 9 ou 10 atomes dans le cycle et 1, 2, 3 ou 4 hétéroatomes dans le cycle, qui sont choisis parmi O, S et N.

4. Composés selon la revendication 1 ou 2, dans lesquels Ar¹ est un groupe phényle sur lequel les groupes A et X sont liés en position méta l'un par rapport à l'autre.

5. Composés selon l'une quelconque des revendications 1, 2, 3 ou 4, dans lesquels Ar² est un groupe phényle.

6. Composés selon l'une quelconque des revendications 1, 2, 3, 4 ou 5, dans lesquels X est un groupe NH.

7. Composés selon l'une quelconque des revendications 1, 2, 3, 4, 5 ou 6, dans lesquels R¹ est un groupe hydroxyle.

8. Composés selon l'une quelconque des revendications 1, 2, 3, 4, 5, 6 ou 7, dans lesquels Y est un groupe de la formule CONH.

9. Composés selon la revendication 1, dans lesquels U est un groupe phényle, substitué le cas échéant.

10. Composés selon la revendication 1 ou 9, dans lesquels V est une liaison directe ou un groupe carbonyle.

11. Composés selon la revendication 1, 9 ou 10, dans laquelle W est un groupe alkyle en C₁ à C₄, un groupe hétéroalkyle en C₁ à C₄, un groupe phényle, substitué le cas échéant, un groupe cycloalkyle en C₃ à C₇, substitué le cas échéant, un groupe hétérocycloalkyle avec 3 à 7 atomes dans le cycle et 1, 2 ou 3 hétéroatomes (choisis parmi O, S et N), substitué le cas échéant ou un groupe hétéroaryle à 5 ou 6 atomes dans le cycle et 1, 2, 3 ou 4 hétéroatomes qui sont choisis parmi O, S et N, substitué le cas échéant.

12. Compositions pharmaceutiques qui contiennent un composé selon les revendications 1, 2, 3, 4, 5, 6, 7, 8, 9, 10 ou 11, et facultativement des substances supports et/ou des adjuvants.

13. Utilisation d'un composé ou d'une composition pharmaceutique selon l'une quelconque des revendications 1, 2, 3, 4, 5, 6, 7, 8, 9, 10, 11 ou 12, à la préparation d'un médicament pour l'inhibition du facteur Xa.

14. Utilisation d'un composé ou d'une composition pharmaceutique selon l'une quelconque des revendications 1, 2, 3, 4, 5, 6, 7, 8, 9, 10, 11 ou 12, à la préparation d'un médicament pour le traitement et/ou la prévention d'affections thromboemboliques, de resténose artérielle, de septicémie, de cancer, d'inflammations aiguës ou d'autres affections qui sont causés par l'intermédiaire du facteur Xa.

15. Utilisation d'un composé ou d'une composition pharmaceutique selon l'une quelconque des revendications 1, 2, 3, 4, 5, 6, 7, 8, 9, 10, 11 ou 12, à la préparation d'un médicament pour des applications en chirurgie vasculaire.
